# EUROPEAN PATENT APPLICATION

(11) **EP 0 791 337 A1**
(43) Date of publication of application: **27.08.1997**
(21) Application number: 97400394.9
(22) Date of filing: 21.02.1997
(51) Int. Cl.: A61B 17/64, A61C 7/10

(54) **Device for mandibular distraction osteogenesis**

(30) Priority: 22.02.1996 US 606039; 22.02.1996 US 606037; 22.02.1996 US 606033
(71) Applicant: Razdolsky, Yan, Buffalo Grove, Illinois 60089 (US); Driscoll, Patrick J., Prospect Heights, Illinois 60070 (US)
(72) Inventor: Razdolsky, Yan, Buffalo Grove, Illinois 60089 (US); Driscoll, Patrick J., Prospect Heights, Illinois 60070 (US)
(74) Representative: Martin, Jean-Paul

(57) **Abstract**

A mandibular distraction device for use in distracting the mandible subsequent to corticotomy thereof includes first and second sets of crowns adapted to be attached to the bicuspid and molar teeth of respective opposite lateral sides of the mandible. A first pair of expander assemblies are removably or fixedly attachable to the opposite sides of the first set of crowns, and a second pair of expander assemblies are removably or fixedly attachable to the opposite sides of the second set of crowns. Each pair of expander assemblies includes one expandable device and one sliding tube assembly. Receptors are provided to be attached to the bicuspid crowns and the molar crowns, and corresponding connectors are provided to be attached to the screw devices and sliding tube assemblies for connection thereof to the crowns.

## Description

### BACKGROUND OF THE INVENTION

The present invention relates generally to the correction of deficiencies in mandibular growth. More specifically, the present invention relates to a device for mandibular distraction osteogenesis the (lengthening of the lower jaw by stretching) for correcting deficiencies in mandibular length.

Deficiencies in mandibular growth which lead to characteristic protrusions of the maxillary teeth and deficiencies of the chin are quite common in American and Northern European populations. Data from recent large scale U.S. Public Health Service surveys of the occlusion of children and youth ages 6 through 10 indicate that about 20 percent of the U.S. population has mandibular deficiency, and about 5 percent of the total U.S. population has skeletal mandibular deficiency (deficiency in the growth of the lower jaw) so severe that the only way to correct such deficiency is to perform a total mandibular (lower jaw) resection (osteotomy) and to advance the lower jaw to a more favorable forward position.

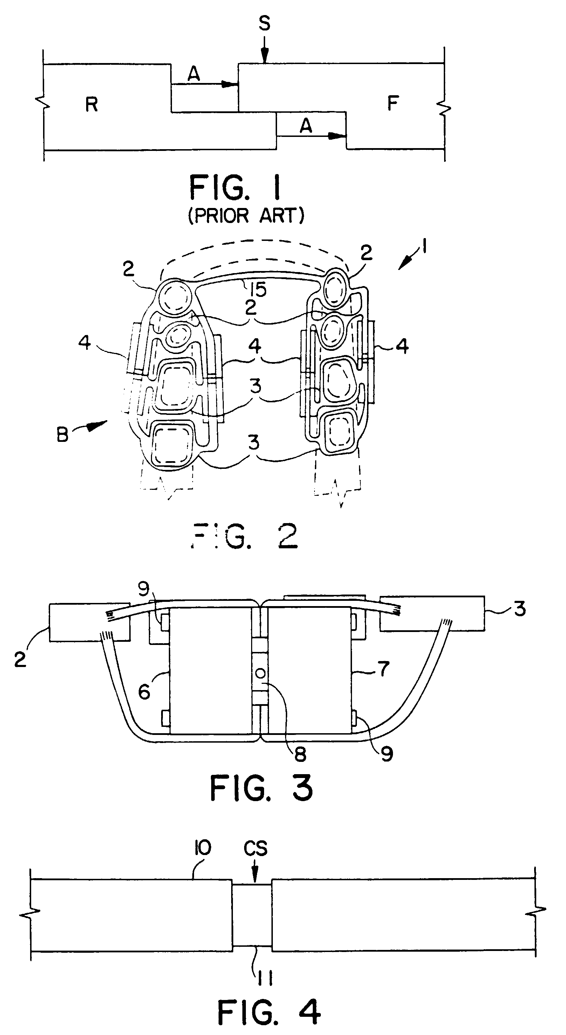

A total mandibular osteotomy, or a sagittal split osteotomy, is a major surgical procedure that can have many complications. In this procedure, as illustrated in Fig. 1, a human mandible is split at opposite points on the mandible. The forward part of the mandible is then brought apart from the rearward part and stabilized with either: (1) screws at point S as labeled in the figure (the forward part F is indicated in Fig. 1 by the arrows A as having been moved; this procedure is used less commonly now than in previous years due to the inherent difficulty in positioning of three loose parts of the mandible correctly during the surgery) or (2) splinting of the broken lower jaw to a prefabricated interocclusal splint which is secured to the upper jaw and allowing it to heal for approximately 2 months (during which the patient cannot open his/her mouth, cannot communicate or function and is fed through a straw).

This procedure cuts the bone marrow, and thus could be detrimental to the inner nerves and blood vessels of the mandible.

In addition, a total mandibular osteotomy can involve the complications of bleeding, obstruction of the airway, possible infection, neurological problems such as possible paralysis of the inferior alveolar nerve and loss of sensation to the lip, failure of intermaxillary fixation (stabilization of the mandible after surgery), relapse-movement of the lower jaw in the direction from which it was advanced, and possible displacement of the temporomandibular jaw joints during the surgery.

Needless to say, such surgery requires a hospital stay, is very expensive and many patients are reluctant to agree to this. Further, total treatment time is on the order of 30 months.

The other 15 percent of mandibular deficiencies are less severe, and if they are caught early, during the pubertal growth stage, are amenable to conventional orthodontics (braces) or a combination of orthodontics and functional appliance treatment. However, functional appliances are of most benefit to a patient when the patient is undergoing body and jaw growth, and cannot benefit adult (non-growing) patients.

One other prior art surgical technique bears mention. A process of lengthening human long bones (limbs, arms, etc.) by distraction osteogenesis has been utilized for the past 40 years. This process was designed by a Russian surgeon, Dr. Gavriel A. Ilizarov. The principles of the method of Dr. Ilizarov are presented in an article based on a speech delivered by Dr. Ilizarov on October 30, 1987 at the annual Scientific Program of the Alumni Association and material presented by Dr. Ilizarov at a three day international conference on the Ilizarov techniques for the management of difficult skeletal problems. His technique is being widely used by orthopedic surgeons throughout the United States and the world.

### SUMMARY OF THE INVENTION

The object of the present invention is to provide an appliance or device suitable for distraction osteogenesis that is applicable to the five percent of severe cases requiring surgery as well as to the less severe 15 percent of cases if those cases have missed their opportunity for orthodontic/functional correction during their pubertal growth years. Distraction osteogenesis is, by definition, the process of generating new bone by stretching. Thus, it is the more specific object of the present invention to provide a device for generating new mandibular bone by stretching the mandible, while orthodontically lengthening the mandible and minimizing the extent of the conjunctive lower jaw surgery.

The objects of the present invention are met by a device used in a method of mandibular distraction osteogenesis. This method involves performing corticotomy surgery, where only the cortex of the mandible is cut, leaving all bone marrow, nerve and blood vessels intact, at two points on opposite sides of the mandible. The device is an expandable distraction device attached to the teeth of the mandible on opposite sides of the two points of the corticotomy surgery, and the expandable distraction device is then periodically expanded until a desired mandibular length is attained.

The method further comprises preparing the expandable distraction device for attachment during the corticotomy surgery. This includes fitting a plurality of stainless steel crowns onto the teeth of the mandible, taking an impression of the teeth of the mandible, removing the crowns from the teeth of the mandible and placing the crowns in the impression, preparing a solid model of the teeth of the mandible from the impression, the crowns being located on the solid model, and then attaching the expansion screw devices to the crowns.

The impression is preferably a rubber base impression that is poured up with dental stone or plaster. The expansion screw devices and sliding tube devices are preferably soldered to the crowns by means of sliding (removable) attachments, in a very precise three dimensional location.

The expandable distraction device is subsequently expanded in the desired direction of the distraction of the mandible. The screw devices separate the crowns on each side of the mandible from each other at the point of the corticotomy surgery on the mandible. Preferably, the expandable screw devices are expanded at a rate of lmm per day, starting the day of the surgery.

The oral ostedistraction device of the invention includes the features mentioned in claim 1.

The objects of the present invention are thus met by the provision of a mandubular distraction device having first and second sets of tooth crowns (or possibly bands), a first set of expandable screw and tube devices connected to the first set of tooth crowns and a second set of expandable screw and tube devices connected to the second set of tooth crowns.

Each expandable screw device preferably comprises first and second body portions that have aligned threaded holes extending therein and a threaded shaft engaging both of the threaded holes.

Further, each set of tooth crowns is preferably disposed along a respective tooth line, each expandable screw device of each set of expandable screw and tube devices being disposed on a side of the tooth line opposite the other sliding tube of the set. Each set of expandable screw and tube devices is soldered to their respective set of tooth crowns.

Further, each set of tooth crowns preferably comprises a plurality of crowns that are aligned for disposition on the teeth of one side of the mandible. Each screw device has a forward portion that connects to some of the bands on one set of tooth crowns and a rearward portion connected to the remainder of the crowns of the set. The forward and rearward portion are thus expandable relative to each other for separation of the crowns from each other.

Preferably, the first set of tooth crowns, having the first set of expandable screw and tube devices thereon, is connected to the second set of tooth crowns, having the second set of expandable screw and tube devices thereon, at forward portions of the expandable screw devices. If so desired, the forward portions of the expandable screw and tube devices can be connected to each other by heavy gauge stainless steel wire.

According to a further preferred feature of the present invention, the first and second sets of tooth engagement members have receptors attached thereto, and the first and second pairs of expander assemblies have connectors attached thereto that are removably engageable with the receptors. Each of the receptors preferably comprises a metal member fixed to at least one of the tooth engagement members and has a connector guide extending thereon, and each connector preferably comprises a metal member fixed to one of the extender assemblies and has an engagement surface complementary to the connector guide of a respective one of the receptors for removable engagement therewith.

The receptors and connectors are preferably made of stainless steel and soldered to the tooth engagement members and the expander assemblies. As noted earlier, the tooth engagement members may be stainless steel bands, but according to a preferred feature of the present invention are stainless steel crowns.

Each expandable screw device comprises first and second body portions having aligned threaded holes extending therein in a threaded shaft engaging both of the threaded holes. Each set of tooth engagement members comprises a plurality of members aligned for disposition on the teeth of one side of the mandible, and each expandable assembly comprises a forward portion connected to some of the members of one set of tooth engagement members and a rearward portion connected to the remainder of the members of the one set of tooth engagement members. The forward and rearward portions are expandable relative to each other for separation of some of the members from the remainder of the members. The first set of tooth engagement members having the first pair of expandable assemblies thereon are connected to the second set of tooth engagement members having the second pair of expandable assemblies thereon at forward portions of the expandable assemblies. The forward portions can be connected by a further expandable assembly, or, as noted above, by heavy gauge stainless steel wire.

The tooth engagement member is comprised of bicuspid and molar engagement members. The receptors preferably include bicuspid receptors attached to the bicuspid engagement members and molar receptors attached to the molar engagement members.

According to the present invention, the invention also contemplates an assembly kit made up of the components described above for constructing a mandibular distraction device and the method of making the mandibular distraction device, it being understood that the mandibular distraction device is a custom made device made by a doctor from the components for a particular patient for the purposes of conducting the above-described procedure.

Through the employment of mandibular distraction osteogenesis according to the present invention, and the use of the mandibular distraction device according to the present invention, a number of significant advantages may be achieved. As noted above, the invention will orthodontically lengthen the mandible while minimizing the extent of the conjunctive lower jaw surgery. Only corticotomy is employed.

Further, the invention will improve the facial profile by advancing or lengthening the deficient mandible. This will improve the lip balance, lip competence, and lip seal. This will also help to eliminate mouth breathing pattern problems. Further, incisor guidance and function will be established.

The invention will also reduce the orthodontic-surgical treatment time. Treatment time can be expected to be reduced to on the order of 12 months, instead of 30 months as with the prior art sagittal split osteotomy surgery.

The invention will also bring the mandible forward, thus bringing the tongue forward and diminishing chances for obstructive sleep apnea or snoring. Such correction will also help to prevent class II mandibular deficiency/malocclusion. The invention will help to correct unilateral cross bites and the mandibular midline.

Further, the invention will minimize damage to the periosteal and endosteal blood supply by performing a corticotomy only, rather than a complete osteotomy as is now performed with the sagittal split osteotomy surgery. This will minimize swelling and post-surgical complications, and requires no hospital stay and could be done on an outpatient basis. Furthermore, the fact that the expansion screw assemblies are detachable from the receptor assemblies means that the orthodontist will generally not need to be present during surgery. The precise pre-alignment will have been done during fitting in the office.

Further, the procedure will be far less expensive than the conventional mandibular osteotomy surgery. Patient costs for the procedure are lower than the costs associated with prior art methods such as the sagittal split osteotomy surgery. Obviously this will tend to lower health care costs in general, which is a great concern at this time.

Other significant advantages result to the benefit of the patient. The procedure according to the present invention results in less pain to the patient than the prior art procedure. The recovery period after completion of the procedure is on the order of two to three days, rather than two months as with other methods. The jaw of the patient does not have to be wired shut for two months, and the patient is able to return to work within one week, as opposed to eight weeks with other methods. Thus it is clear that the psychological impact of the procedure on the patient will be significantly reduced as compared with the impact of the prior art methods.

### BRIEF DESCRIPTION OF THE DRAWINGS

The present invention will be described in detail below with reference to the accompanying drawings, in which:
Fig. 1 is a schematic drawing illustrating sagittal split osteotomy surgery;
Fig. 2 is a top view of a mandibular distraction osteogenesis device according to the present invention;
Fig. 3 is a side view of a portion of the mandibular distraction device as seen in the direction of arrow B of Fig. 2;
Fig. 4 is a schematic representation of corticotomy surgery;
Fig. 5 is a partial perspective view of a Razdolsky attachment plug according to a first embodiment of attachments for the mandibular distraction device according to the present invention;
Fig. 6 is a perspective view of a receptor according to the first embodiment of the Razdolsky attachments, the receptor being mounted on a crown;
Fig. 7 is an exploded view of the plug and a screw device according to the first embodiment of the Razdolsky attachments;
Fig. 8 is an exploded view of a screw device, a cap and a receptor according to a second embodiment of the Razdolsky attachments according to the present invention;
Fig. 9 is a perspective view of a cap and a receptor according to a third embodiment of the Razdolsky attachments of the present invention;
Fig. 10 is a perspective view of the cap of Fig. 9;
Fig. 11 is a perspective view from the rear of the receptor of Fig. 9;
Fig. 12 is a perspective view of a receptor according to a modification of the third embodiment of the Razdolsky attachments;
Fig. 13 is a perspective view of a cap suitable for use with the receptor of Fig. 12;
Fig. 14 is a perspective view of a screw device, cap and receptor according to a fourth embodiment of the Razdolsky attachments of the present invention;
Fig. 15 is a perspective view of the cap of Fig. 14;
Fig. 16 is a perspective view of the receptor of Fig. 14;
Fig. 17 is a schematic view of a receptor according to the fourth embodiment of the Razdolsky attachments used together with the receptor according to a fifth embodiment of the Razdolsky attachments of the present invention;
Fig. 18A is a perspective view of the receptor according to the fifth embodiment of the Razdolsky attachments;
Fig. 18B is a perspective view of a screw device, cap and receptor according to the fifth embodiment;
Figs. 19A-19D are illustrations of steps in the process of using the present invention;
Fig. 20 is a schematic illustration of another embodiment of the distraction device according to the present invention as applied to a mandible;
Fig. 21 is a top view of an alternative arrangement of engagement members; and
Fig. 22 is a schematic view of a paralleling tool for use with the Razdolsky attachments.

### DETAILED DESCRIPTION OF THE PREFERRED EMBODIMENTS OF THE INVENTION

A detailed description of the present invention will now be presented with reference to the accompanying drawing figures. In the various figures, the same reference numerals are used for similar elements throughout. The description of the invention will proceed with the description of a mandibular distraction osteogenesis device and in particular Razdolsky attachments for the purpose of securing expansion screws and sliding tubes onto the stainless steel crowns in a precise angular fashion and making both expansion screws and sliding tubes removable prior to corticotomy surgery.

Turning to Fig. 2, there is illustrated a mandibular distraction osteogenesis device 1 usable in distracting the mandible. Initially, the device 1 includes a plurality of crowns (or bands, collectively also referenced as tooth engagement members) for placement on the teeth of the mandible of a patient that is to undergo distraction osteogenesis. The tooth engagement members of the present invention are preferably crowns, but it should be recognized that bands could also be employed instead of crowns; the description will primarily discuss crowns. In Figs. 2 and 3, while the description references crowns, the illustration in these figures is not meant to be indicative of any particular type of crown but to be simply a generic description of a crown or band for purposes of illustrations.

Preferably there are provided a total of eight crowns, with two bicuspid and two molar orthodontic crowns being provided for each side of the mandible, as illustrated in Fig. 2. The crowns are indicated by reference numbers 2 for the bicuspid crowns and reference numbers 3 for the molar crowns. The mandible and the relevant teeth are schematically illustrated by a dashed line in Fig. 2.

One universal expansion screw 4 is soldered onto each buccal (cheek) side of the crowns and one universal sliding tube device 5 is soldered onto each lingual (tongue) side of the crowns for each side of the mandible. One universal expansion screw 4 and one sliding tube 5 is thus placed on each side of each set of crowns. As can be seen from Fig. 2, the universal expansion screws thus extend along the sides of the crowns and have suitable portions thereof soldered to the respective crowns. The universal expansion screws 4 are expandable to distract a forward portion of the mandible, the upper portion as seen in Fig. 2, from a rearward portion of the mandible by separating the bicuspid bands 2 from the molar bands 3.

More specifically, and referring to Fig. 3, each universal expansion screw 4 has two halves 6 and 7 separable from each other by a screw mechanism 8. The screw mechanism 8 is a suitable mechanism rotatable between the universal expansion screw halves 6 and 7 to separate the halves from each other, such as a right and left hand threaded shaft extending into and engaging with corresponding threads in the halves 6 and 7. Suitable guide rods 9 can also extend through the halves 6 and 7 to guide the separation of the halves 6 and 7 from each other. As can be seen, suitable connecting portions are provided for connecting the halves 6 and 7 to the respective bands 2 and 3. Such connecting portions can take the form of appropriate metal wires or bars. The universal expansion screw 4 can be of the type illustrated in U.S. Patent 4,482,318, for example, or could be of the type shown in U.S. Patent 4,571,177, suitably adapted to the present situation. These patents are incorporated herein by reference.

By the above construction there is formed two separate portions of the mandibular distraction device 1, one portion being located on each side of the mandible. These portions are preferably connected to each other by a suitable connecting wire or bar 15, as illustrated in Fig. 2. However, note that in place of the connecting wire or bar 15, an additional, smaller, universal expansion screw 4 could be provided and incorporated into the device 1, the universal expansion screw connecting the two sides of the device 1 at the forward portions thereof in order to allow for lateral mandibular expansion, in addition to mandibular distraction or elongation.

As can be seen from Fig. 2, the bicuspid crowns 2 on each side of the mandible are connected to the forward portions or halves 6 of the universal expansion screws 4, and the molar crowns 3 are connected to the rear portions or halves 7 of the universal expansion screws 4. Thus, a unitary forward portion is expansible in a forward direction relative to two separate lateral portions on opposite sides of the mandible for elongation or distraction of the mandible.

Though not specifically illustrated, the sliding tubes 5 represent simple expandable sliding tube and pin connections connecting the forward and rearward portions of the device 1 on each side of the mandible. These tube and pin connections have a simple tube receiving a pin with the tube connected to one portion and the pin connected to the other portion and extending in the same direction as the expansion screws 4. Thus these devices will simply follow the distraction of the mandible along with the activation of the screw devices 4, but will provide for support on the lingual side of the crowns in all directions except for the direction of expansion. Such sliding tubes, as well as the expansion screws and crowns, are separately readily available from orthodontic suppliers.

While the above described distraction device 1 simply solders the expansion screws and sliding tubes 5 to the crowns 2 and 3, it is preferred that specific attachments be employed for this purpose, as will be described below. These attachments, generally referred to as Razdolsky attachments, comprise receptor attachments attached to the respective crowns, preferably by soldering, and connector attachments connected to the respective screw devices and sliding tubes 5 also by soldering. The receptors and connectors are thus removably engageable with each other so that they screw devices 4 and sliding tubes 5 can be removably attached to the crowns, for reasons as will be discussed below in describing the method of mandibular distraction osteogenesis in accordance with the device of the present invention. At this point, specific description of the Razdolsky attachments will be made.

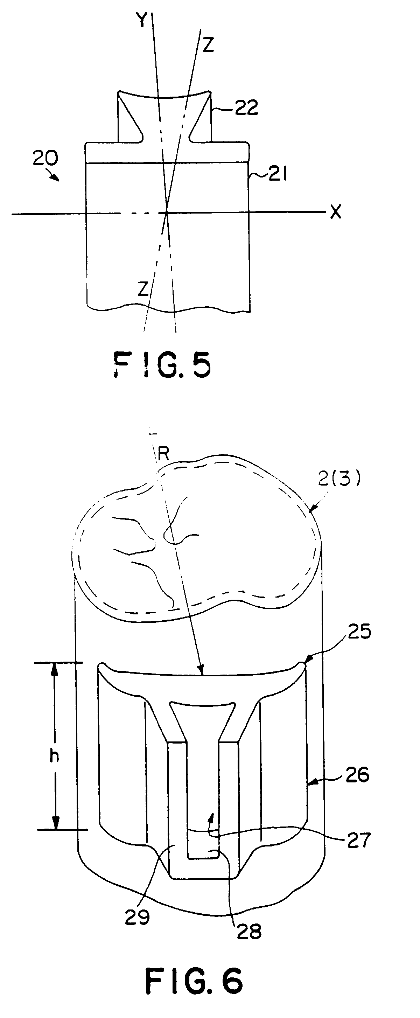

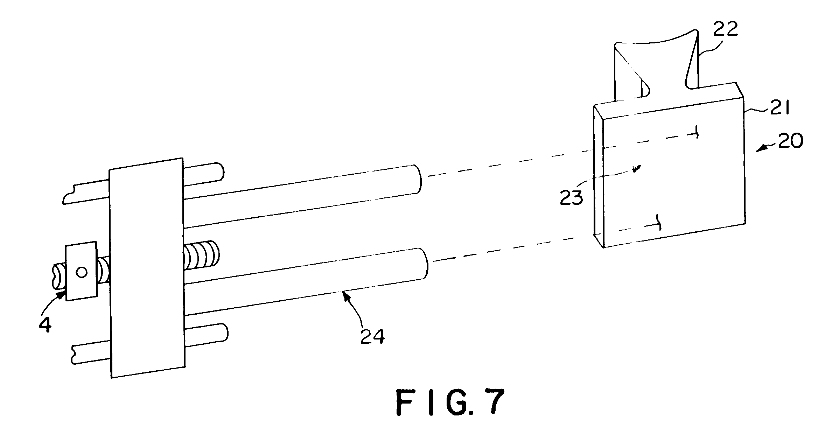

A first embodiment of the Razdolsky attachments is illustrated in Figs. 5-7. Fig. 5 illustrates a plug attachment for attachment to a screw device 4, Fig. 6 illustrates a crown 2 or 3 having a connector 25 connected therewith and Fig. 7 illustrates a connection between the screw device 4 and the plug 20.

The attachment plug 20 according to the Razdolsky attachments comprises a front portion 21 having a front surface and a plug portion 22. The plug portion 22 tapers from its distal end in the Z direction to the front portion 21, as illustrated.

Fig. 6 illustrates a stainless steel crown 2 or 3, preferably a stainless deep drawn thin shell molar or bicuspid cap as are commercially available. The receptor 25 is soldered to the stainless steel cap, and is preferably itself an investment cast stainless steel, etc. Solder flow details are provided on each side as illustrated at 26, noting the beads on the vertical sides of the receptor 25. The majority of the receptor 25 comprises the back portion soldered to the cap. A slot 27 is formed by a front portion 29, which has outwardly jutting walls defining the slot 27 as a slot tapering from the back portion toward the front. The slot is opened at its top and forward sides, and is closed at the back and lower portions thereof. The lower portion at 28 forms a vertical stop.

As seen in Fig. 7, expansion device solder legs 24 of the screw device 4 can be soldered to the front portion 21 of the plug 20 as noted at 23, designating a solder surface on the plug 20. During assembly, the plug 20 can then have its plug portion 22 vertically inserted into the slot 27 of the receptor 25, the plug 22 being complementary to the slot 27 for a snug fit. The plug 22 can be a solder plug and can provide a snug fit with a very low viscosity adhesive joint in the receptor 25.

The receptor 25 has a height h and a radius of its back surface R. The receptor 25 can thus be provided in several general ranges of sizes for general ranges of the sizes of teeth.

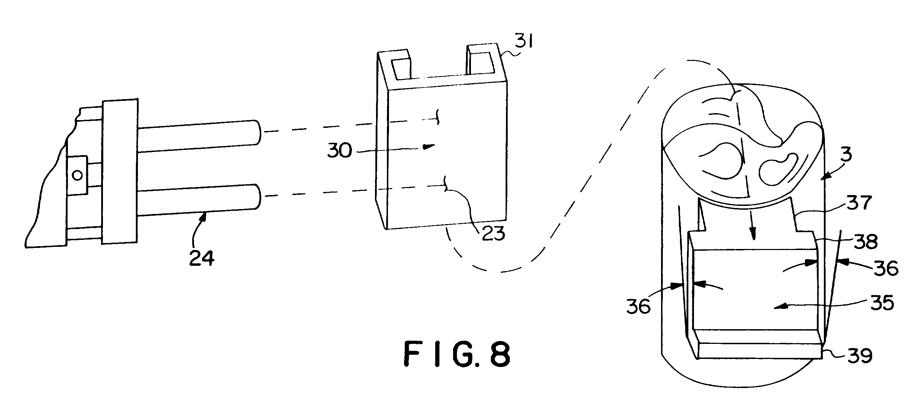

A second embodiment of the Razdolsky attachments is illustrated by Fig. 8. In this figure, the attachments comprise a cover or cap 30 in place of the plug, and a receptor 35. The receptor 35 is illustrated as attached to a molar crown 3, for example by soldering. The receptor 35 has a front portion 38 having lateral flanges with respect to the back portion 37 that is connected to the crown 3. The flanges have a slight taper shown at 36. The cap or cover 30 has flanges or channel members 31 forming channels for engagement with the flanges of the front portion 38. When engaged as illustrated, the slight taper wedges the cap or cover 30 in place, and the cap 30 engages a vertical stop 39 on the bottom of the receptor 35.

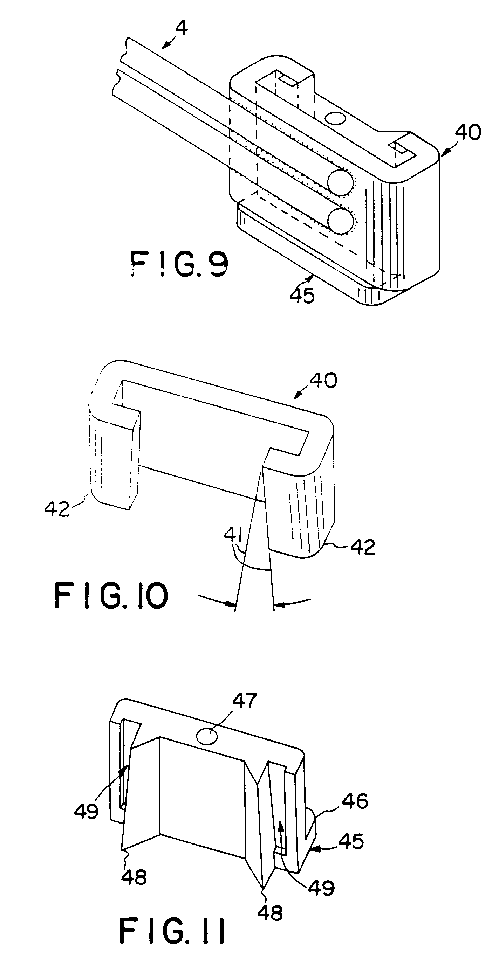

Figs. 9-11 illustrate a third embodiment according to the Razdolsky attachments of the present invention. In this embodiment, the cap 40 engages a receptor 45. The receptor 45 has a vertical stop 46 similar to the above embodiments, and an alignment hole 47. The cap 40 has channels 42 for engaging the rear surface of the receptor 45, with the turned flanges of the channels 42 having a taper at 41. The taper is provided for a tight fit against triangular engagement members 48 on the receptor 45, which are similarly tapered. According to a particular feature of this embodiment, glue pockets 49 are provided on the back surface of the receptor 45 for gluing the cap 40 to the receptor 45 during surgery.

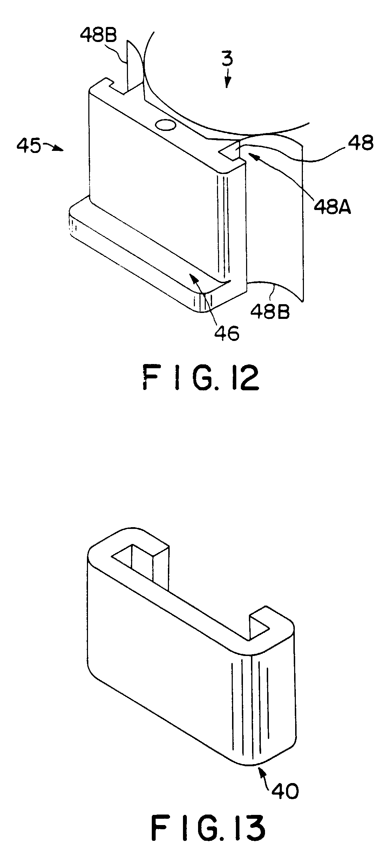

In a variation of the third embodiment illustrated in Fig 12, bendable wings 48b can be soldered at 48a to the rear engagement portions 48 of the receptor 45. These bendable wings can engage a molar cap 3.

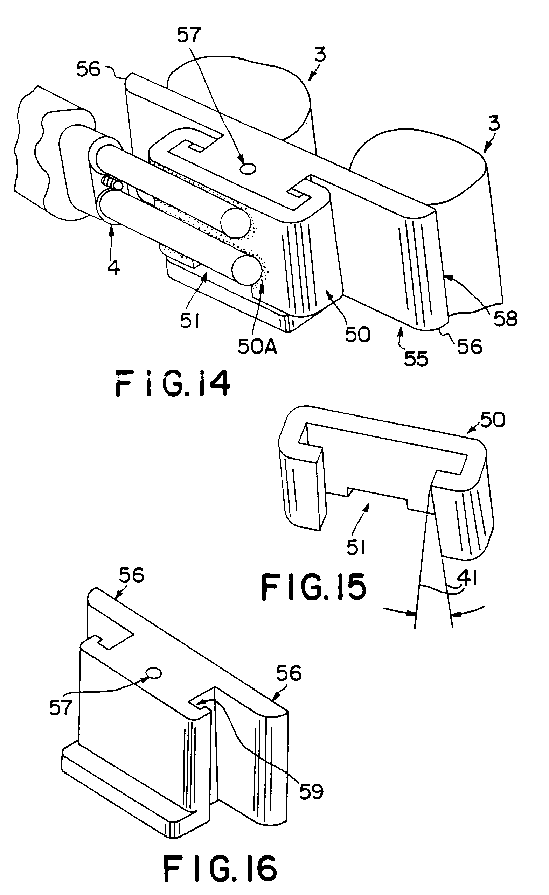

Fig. 13 provides a front perspective view of the cap 40 according to the third embodiment of the Razdolsky attachments of the present invention.

Figs. 14-16 describe a fourth embodiment of the Razdolsky attachments according to the present invention. A cap 50 is similar to the cap discussed with respect to the third embodiment of the Razdolsky attachments and is connected to the screw device 4 in a similar manner. However, in this embodiment a slot 51 is provided in the cap for receipt of a pry bar for removal of the cap from the receptor during an intermediate step of the procedure. It will be recognized that the slot could be provided with the other embodiments of the connectors of the present invention. A receptor 55 of this embodiment is similar to the receptor of the third embodiment of the Razdolsky attachments in that it has a similar front portion providing a vertical stop and an alignment hole 57, and provides similar glue pockets at the rear surface of the front portion. However, with this embodiment the rear portion is extended further back and connected with two laterally extending tabs 56 so that the single receptor 55 may be connected with two molar caps 3 as illustrated in Fig. 14. As seen at 58, the tabs 56 are soldered to the stainless steel caps. Thus employment of this embodiment will reduce the number of receptors and caps necessary for connection of the expansion screw devices 4 and sliding tube devices 5 on the sides of the lines of crowns. This will be further discussed below.

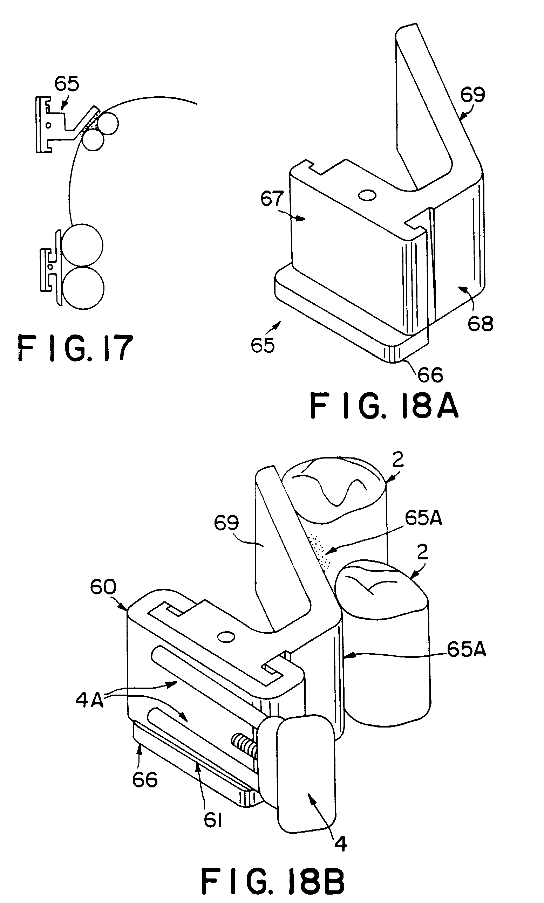

Figs. 17-18B illustrate a fifth embodiment of the Razdolsky attachments according to the present invention, and can be used together with the fourth embodiment. That is, in this embodiment a receptor 65 has a front portion 67 with a vertical stop 66 similar to that of the third and fourth embodiments. However, instead of having the laterally extending tabs of the fourth embodiment, an intermediate portion 68 extends rearwardly from one side of the rear surface of the front portion 67, and continues into a back portion 69 that extends from the intermediate portion 68 at an angle that is acute relative to the direction of the expander assemblies. The cover 60 is similar to the previous embodiments and is provided with a pry slot 61 for engagement by a suitable tool, such as a thin bladed screwdriver, etc. As illustrated in Fig. 18B, the back portion 69 is soldered at 65a to two bicuspid crowns 2. The angle of the portion 69 allows the front 67 to be better aligned with respect to the assembly of the screw device 4 with its respective caps soldered thereto. In this regard, note Fig. 17. In this figure, two bicuspid caps are seen as connected with the receptor 65 and two molar caps are seen as connected with the receptor 55 according to the fourth embodiment. As seen in the figure, by the angled rear portion 69 of the receptor 65, both the front portions of the respective receptors can be aligned with reach other, making the process of assembly a simple matter.

With respect to the fifth embodiment, the bicuspid receptor 65 that is illustrated in Fig. 18A is obviously only usable on one side of the distraction device, i.e. on one side of the mandible. However, it is contemplated that a symmetric bicuspid receptor could be manufactured that would be usable on both sides of the mandible so that only one part would have to be manufactured.

The method of mandibular distraction osteogenesis according to the present invention is as follows. Referring to Figs. 2-4, first two bicuspid and two molar orthodontic crowns are fitted onto the respective teeth of a patient's mandible on each side of the mandible. Thus, a total of eight crowns are fitted onto the teeth of the patient. A rubber base impression is then taken of the patient's mandible with the crowns in place. The crowns are then removed and placed into the impression. Then, the impression is poured up with orthodontic (dental) stone or plaster, so as to form a model of the patient's mandible, with the crowns in place thereon on the appropriate teeth of the mandible model.

The two universal expansion screws 4 are then soldered onto the connectors of the Razdolsky attachments and the receptors are soldered onto the crowns (Fig. 19A) in a very precise angular fashion preferably using the laboratory instrument discussed in U.S patent 5,559,183 (incorporated herein by reference). Two sliding tubes are also soldered onto the crowns 2 and 3 in a simple fashion utilizing the Razdolsky attachments. Thus the mandibular distraction device 1 is formed. A suitable connection 15 (Fig. 2, which shows the embodiment not using the attachments) may also be provided, or an additional universal expansion screw 4 may also be provided in place thereof to provide for lateral mandibular expansion. With the finished mandibular distraction device 1, the device is now ready to be cemented into the patient's mouth.

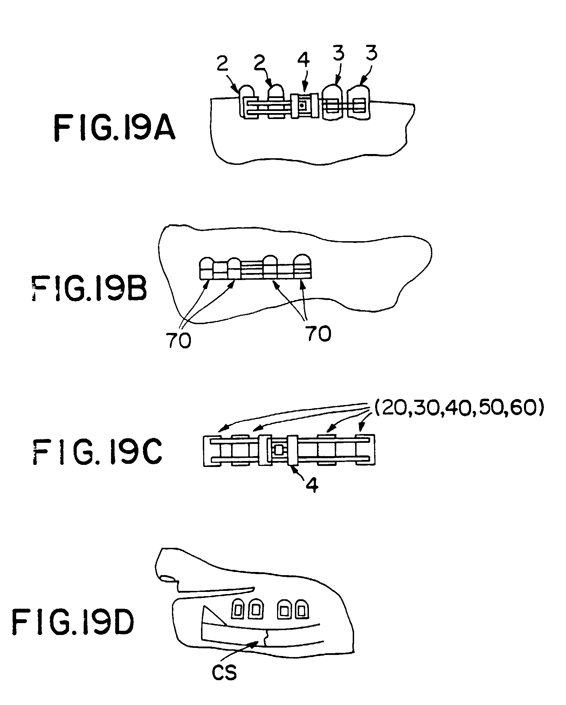

Accordingly, the mandibular distraction device is cemented into the patient's mouth prior to corticotomy surgery as at 70 (Fig. 19B). All expansion screws and sliding tubes are then removed (Fig. 19C) by means of the Razdolsky attachments, which guarantee the previous exact angular positioning, and are only to be reinserted after the corticotomy surgery is performed. When the screw devices and sliding tubes are removed by the use of the Razdolsky attachments, the crowns stay cemented on the patient's teeth. This technique provides for maximum access and visibility during the surgery. Corticotomy surgery is then performed, which is the cutting of the outside layer (cortex) only of the mandible (Fig. 19D). Referring to Fig. 4, a section of the patient's mandible is schematically illustrated. Portion 10 represents the outer layer of the bone, i.e. the cortex. This portion is cut during the corticotomy surgery. However the bone marrow 11 is left intact. This reduces the chance of the nerves or the blood vessels being severed. The location of the corticotomy surgery is represented in Fig. 4 by the letters CS. The corticotomy surgery is performed at two points on opposite sides of the mandible to allow for the elongation or distraction of the forward portion of the mandible from the rearward portion thereof. The corticotomy preferably takes place posterior to the lower second bicuspids, and preferably between the bicuspids and the molars on each side of the mandible to allow for the two bicuspid crowns 2 on each side to be displaced forwardly from the rear molar crowns 3 with the expansion of the universal expansion screws 4. Appropriate x-rays can be taken of the mandible in order to determine the exact thickness of the cortex to ensure that the bone marrow 11 is not cut.

After the reinsertion of the expansion screws and sliding tubes after corticotomy surgery, the mandible is then distracted by expanding the two universal expansion screws 4 inside of the patient's mouth. This is accomplished by rotating the screws 8 of the universal expansion screws 4 periodically to extend the forward portion of the mandible from the rearward portion thereof. This is possible because the cortex has been cut in the corticotomy surgery. The bone marrow is softer tissue and allows for elongation to take place. Both bone and soft tissue regeneration will occur during the process of expanding the universal expansion screws 4 and distracting the mandible. The mandible can be distracted at a rate of lmm per day until the proper mandibular length is obtained. Recommended distraction is at a rate of 0.5mm to 1.0mm per day until the proper length is obtained. The appliance is left in place two days for each lmm of expansion in order to allow for the complete bony union after expansion. There may be differential expansion between the left and right sides in order to maintain expansion along the centerline of the mandible.

Note that when reinserting the expansion screws and sliding tubes, the respective connectors are connected with the respective receptors. At this time, the receptors and connectors are preferably permanently bonded to each other prior to adjustment of the mandible by a suitable adhesive. However, as an alternative to adhesive, it is contemplated that a locking mechanism could be provided between each connector and receptor. Such a locking mechanism would preferably be of a type in which the receptor and connector are securely fixed with each other, but which could be quickly released by the orthodontist or surgeon, and different types of such locking mechanisms will occur to those of skill in the art.

Variations on the above method and devices are further contemplated. For example, in Fig. 2 above four expander assemblies 4 were illustrated for use with the distraction device, one expander assembly being disposed on each side of each line of tooth engaging members 2 and 3. A screw assembly does not need to be on each side in accordance with the above discussion, and could be replaced by a simple slide assembly. However, the slide assembly could also be removed entirely. That is, it would be sufficient in terms of the proper functioning and strength of the distraction device as a whole if a single screw assembly was employed on each side of the distraction device, with no other support on the opposite side of thetooth line such as was before provided by the slide assembly. Thus, for example looking at Fig. 2, the two inside expander assemblies 4 could be removed entirely, with no other support on that side, and sufficient strength and support be maintained.

In another variation according to the present invention, it is particularly noted that the engagement members are not limited to eight tooth engagement members as illustrated in Fig. 2. That is, instead of having two tooth engagement members on each side of the corticotomy site on each side of the jaw as illustrated in Fig. 2, a single tooth engagement member could be provided on each side of the corticotomy site. In this instance, it is preferred that additional support be provided. This will be explained in the following.

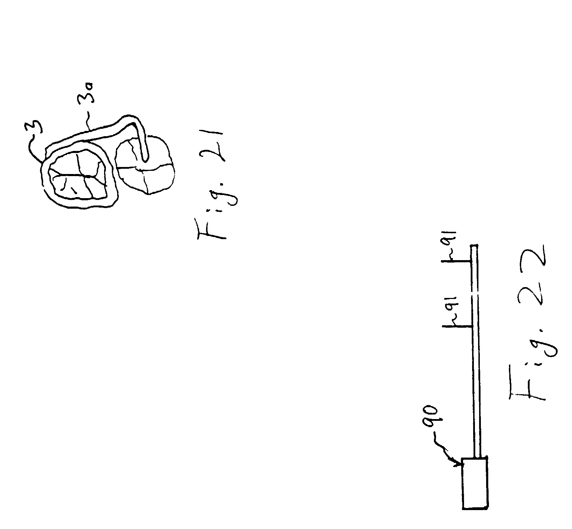

For example, a molar cap 3 is illustrated in Fig. 21 on top of a molar, adjacent to an un-capped tooth, for example another molar. A wire or extension 3a can extend to the adjacent tooth, extending along the side thereof in close proximity thereto, and up and into the center portion of the tooth to provide additional support beyond the single crown or cap. When this arrangement is used, it is preferred that the tooth adjacent to the corticotomy site be uncapped and have the wire 3a extending thereto, and that the cap be placed on the next tooth in line. Thus, an arrangement could be provided only employing a total of four molar caps 3, for example, each having a respective wire or extension 3a to the teeth adjacent to the corticotomy site. Thus, if reference is again made to Fig. 2, the two inner caps 2 and 3 on each side of the jaw could be replaced by wire extensions leading from the outer caps 2 and 3. This can be combined with the removal of the interior expander assemblies 4 to provide a very open and simple arrangement, yet one having sufficient strength in operation.

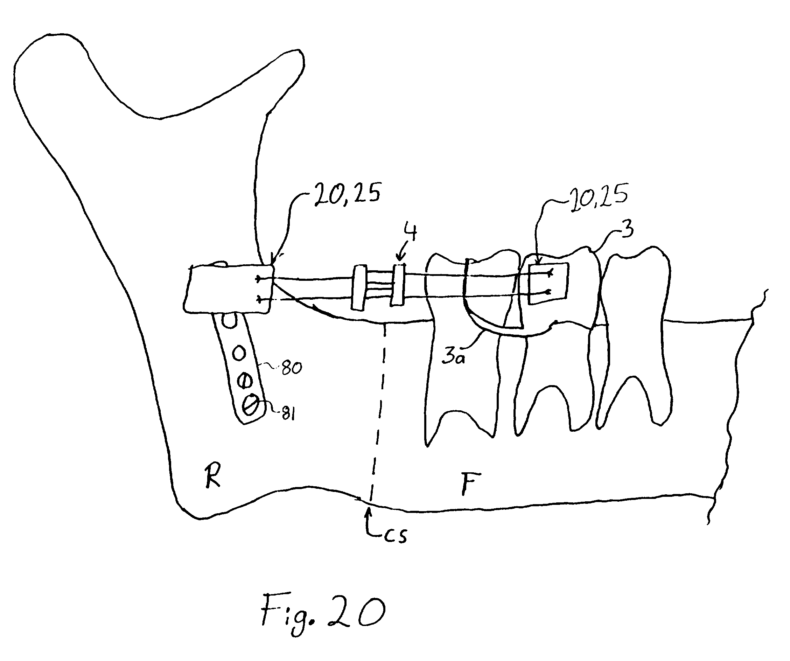

Referring now to Fig. 20, an alternative arrangement of a distraction device is illustrated. In this arrangement, while only one side of the jaw is illustrated, a similar arrangement is provided for the other side of the jaw.

As can be seen from Fig. 20, the concept here is to perform corticotomy at a site rearward of the last tooth along the tooth line. This involves not having to make the corticotomy between two teeth. The extension of the mandible thus takes place rearward of the last tooth, in some respects simplifying the overall operation.

The components necessary for this device can be similar to those above, or can be according to the preferred arrangement in Fig. 20. In Fig. 20, on one side of the mandible, on the forward portion F, a single molar crown 3 is provided. A wire attachment 3a extends from this crown 3 to the adjacent tooth. As discussed above, the wire extension 3a extends toward the corticotomy site CS. Rearward of the corticotomy site, a bone plate 80 is attached by bone screws 81 at a suitable location for purposes of anchoring the attachments used in connecting the screw expander assembly 4. A suitable receptor 25 is welded to the bone plate 80, and a suitable receptor 25 is welded to the crown 3, as discussed above. A screw expander assembly is connected to the connectors 20 as also discussed above, the connectors 20 being connectable with the receptors 25 for use in distraction.

The distraction procedure discussed above can also be performed with the maxilla. In a preferred arrangement, the preformed stainless steel crowns are placed over the second molars on the first bicuspids, but other combinations will also work, for example using the second bicuspid and the first molar, etc. The second molar and the first bicuspid are preferred, because the osteotomy is performed between the second bicuspid and the first molar and the crowns on the second molars and the first bicuspid thus do not interfere with the surgery (osteotomy).

In maxillary distraction, the stainless steel crowns could be placed anywhere, depending on the area of the osteotomy and the desired amount of distraction.

As discussed above, in the method according to the present invention a rubber base impression is taken of the mouth with the stainless steel crowns in place. The crowns are then removed from the mouth and placed in the impression, pinned into position, and the impression is poured up with green or any heat resistant dental stone or material. A model is then produced with the stainless steel crowns on it. After this step, the removable attachments are soldered to the stainless steel crowns. In order to maintain the attachments aligned, the vertical holes 57 in the attachments come into play. That is, a paralleling tool 90 as illustrated in Fig. 22 has two parallel pins thereon inserted into the holes 57 of the two receptors used for a particular side of the jaw. It is very important that these receptors be maintained parallel during soldering so that the expander assembly 4 can be properly attached and detached. Thus the paralleling tool 90 is used to align the attachments during soldering. Note that the pins 91 are adjustable along the shaft of the tool for different desired spacings between the respective attachments.

The model having the attachments thereon is then placed into a laboratory tool, for example the tool discussed above with respect to U.S. patent 5,559,183. This tool is used to solder the expanders bilaterally correctly into the proper three dimensional position as determined from the skull, panoramic X-rays and study models or other diagnostic materials as available.

Once the expanders are soldered, two separate orthodontic wires as wires 3a illustrated in Fig. 20 are soldered on the lingual surfaces of the mandibular second molar and mandibular first bicuspid (in this example) and adapted to the occlusal surfaces of the mandibular first molar and the second bicuspid. The wire that is adapted to the first molar and the second bicuspid will later be bonded to the respective teeth in the mouth.

The distraction device is now ready to be cemented into the patients mouth. The device or appliance is cemented via the crowns to the second molars and the first bicuspids (in this example). The lingual wires which were soldered to the lingual surface of the second molar and first bicuspid crowns are now bonded to the occlusal surface of the mandibular first molar and the second bicuspid. The two expanders 4 are then removed by means of the removable attachments in order to allow the osteotomy surgery to take place. The expanders are reattached by the attachments after surgery and are secured in place with orthodontic or surgical wire (as an alternative to the above-referenced glue or locking) through the vertical holes 57 in the attachments.

While a wire is used, in the above described embodiment, to connect the receptors and the connectors 25 and 20, a locking device could be incorporated into the attachments. For example, a releasable coupling between the receptors and connectors could be provided. As an alternative, clips could also be provided for simply clipping the connectors and receptors. In terms of mechanical play, however, either adhesive or twisted alloy wire extending through the vertical holes 57 is preferred. However, hardened stainless wire clips for retaining the receptors and connectors after surgery could be contemplated if crimped tight with surgical pliers. Also, the orthodontic wire could be used to secure the expander in place after surgery, and in addition, it could be glued or bonded over for additional stability.

While preferred embodiments of the present invention have been described above in some particularity, the scope of the present invention should not be limited thereby, as various modifications thereof will be apparent to those of skill in the art.

## Claims

1. An oral osteodistraction device, comprising:
first and second sets of engagement members adapted to be attached to teeth and/or bone of respective opposite lateral sides of one of the bones of the jaw;
a first expander assembly attached to said first set of engagement members, said first expander assembly comprising at least one expandable screw device; and
a second expander assembly attached to said second set of tooth engagement members, said second expander assembly also comprising at least one expandable screw device.

2. The distraction device of claim 1, wherein said first and second sets of tooth engagement members have receptors attached thereto, and said first and second expander assemblies have connectors attached thereto that are removably engageable with said receptors.

3. The distraction device of claim 2, wherein:
each of said receptors comprises a metal member fixed to at least one of said engagement members and has a connector guide extending thereon; and
each said connector comprises a metal member fixed to one of said expander assemblies and has an engagement surface complementary to said connector guide of a respective one of said receptors for removable engagement therewith.

4. The distraction device of claim 3, wherein:
at least one of said receptors comprises a back portion fixed to at least one of said tooth engagement members and a front portion defining said connector guide, said connector guide comprising a vertical slot open at front and upper areas thereof and closed at said back area and a lower area thereof, said lower area defining a vertical stop, and said slot tapering from said back area adjacent to said back portion toward said front area; and
at least one of said connectors comprising a plug having a front portion fixed to one of said expander assemblies and a plug portion that extends vertically and has a taper complementary to said vertical slot of said at least one of said receptors.

5. The distraction device of claim 3, wherein:
at least one of said receptors comprises a back portion fixed to at least one of said tooth engagement members and a front portion defining said connector guide, said connector guide comprising a vertically tapering front member having a pair of vertically extending flanges thereon defining spaces between said front portion and said tooth engagement members and a vertical stop; and
at least one of said connectors comprising a cap having a front portion fixed to one of said expander assemblies having a rear surface engageable with said front portion of said at least one of said receptors and channel members on said front portion that extend vertically and have a taper complementary to said vertically tapering front member of said receptors, said front portion being engageable with said vertical stop.

6. The distraction device of claim 5, wherein:
said front portion of each of said receptors has a vertical alignment hole extending therein from an upper surface thereof, wherein said vertical alignment holes of each of said receptors corresponding to one of said first and second expander assemblies are parallel with each other.

7. The distraction device of claim 5, wherein said cap has a slot extending horizontally across said front portion thereof at a lower end of said cap.

8. The distraction device of claim 5, wherein said back portion of said at least one of said receptors comprises horizontally extending tabs and said back portion is fixed to two of said tooth engagement members.

9. The distraction device of claim 3, wherein said receptors and said connectors are made of stainless steel and soldered to said tooth engagement members and said expander assemblies, respectively.

10. The distraction device of claim 1, wherein said tooth engagement members are stainless steel crowns.

11. The distraction device of claim 1, wherein said tooth engagement members are stainless steel bands.

12. The distraction device of claim 1, wherein each said expandable screw device comprises first and second body portions having aligned threaded holes extending therein and a threaded shaft engaging both said threaded holes.

13. The distraction device of claim 1, wherein each said set of engagement members comprises a plurality of members aligned for disposition on the teeth of one side of the mandible, and each said expander assembly comprises a forward portion connected to some of said members of one said set of engagement members and a rearward portion connected to the remainder of said members of the one said set of engagement members, said forward and rearward portions being expandable relative to each other for separation of said some of said members from said remainder of said members.

14. The distraction device of claim 13, wherein forward portions of said expander assemblies and some of said engagement members are connected to each other by a third expander assembly.

15. The distraction device of claim 3, wherein:
at least one of said receptors comprises a front portion defining said connector guide, said connector guide comprising a pair of vertically extending flanges, a vertical stop on said front portion, an intermediate portion extending from said front portion and a back portion extending from said intermediate portion and fixed to at least one of said tooth engagement members, said intermediate portion spacing said back portion from said front portion; and
at least one of said connectors comprising a cap having a front portion fixed to one of said expander assemblies, a rear surface engageable with said front portion of said at least one of said receptors and channel members on said front portion for engagement with said flanges, said front portion being engageable with said vertical stop.

16. The distraction device of claim 1, wherein each of said first and second expander assemblies comprises a single said expandable screw device on one side of the respective said set of engagement members and an expandable tube and pin slider device on the other side of the respective said set of engagement members.

17. The distraction device of claim 1, wherein each of said first and second sets of engagement members comprises at least one tooth engagement member and one bone engagement member, and wherein said first and second expander assemblies are removably attached to said first and second sets of engagement members by receptors fixed to said first and second sets of engagement members and connectors fixed to said first and second expander assemblies, said connectors being removably engageable with said receptors.

18. The distraction device of claim 17, wherein said bone engagement member comprises a bone plate and bone screws for fixing said bone plate to a bone of the jaw, and one of said receptors is fixed to said bone plate.

19. The distraction device of claim 1, wherein each of said first and second sets of engagement members comprises at least two tooth engagement members for disposition on either side of a corticotomy site.

20. The distraction device of claim 19, wherein each of said at least two tooth engagement members for disposition on either side of a corticotomy site comprises at least a first tooth engagement member for disposition on a tooth on one side of the corticotomy site and at least a second tooth engagement member for disposition on a tooth on the other side of the corticotomy site, wherein at least one of said first and second tooth engagement members comprises a wire extension fixed thereto for extending to and engaging an adjacent tooth.

21. Attachments for use with an oral osteodistraction device, comprising:
a receptor attachment adapted to be connected with an engagement member of an osteodistraction device, said receptor comprising a first metal member having an engagement member connection portion and a connector receiving portion; and
a connector attachment adapted to be connected with an expander assembly of an osteodistraction device, said connector attachment comprising a first metal member comprising an expander assembly connection portion and a receptor connection portion that is engageable with said connector receiving portion of said receptor attachment;
wherein said connector receiving portion of said receptor attachment comprises a connector guide extending thereon; and
wherein said receptor connection portion of said connector comprises an engagement surface complementary to said connector guide of said receptor for removable engagement therewith.

22. The attachments of claim 21, wherein said receptor comprises an alignment surface thereon for use in aligning said receptor attachment during fixation with the engagement portion.

23. The attachments of claim 21, wherein:
said first metal member comprises a back portion defining said tooth engagement member connection portion and a front portion defining said connector guide, said connector guide comprising a vertical slot open at front and upper areas thereof and closed at a back area and a lower area thereof, said lower area defining a vertical stop, and said slot tapering from said back area adjacent to said back portion toward said front area; and
said second metal member comprises a plug having a front portion defining said expander assembly connection portion and a plug portion that extends vertically and has a taper complementary to said vertical slot of said first metal member.

24. The attachments of claim 21, wherein:
said first metal member comprises a back portion defining said tooth engagement member connection portion and a front portion defining said connector guide, said connector guide comprising a vertically tapering front member having a pair of vertically extending flanges thereon, defining spaces between said front portion and said tooth engagement member connection portion, and a vertical stop; and
said second metal member comprising a cap having a front portion defining said expander assembly connection portion and a rear surface engageable with said front portion of said first metal member and channel members on said front portion of said second metal member that extend vertically and have a taper complementary to said vertically tapering front member of said connector guide, said front portion of said second metal member being engageable with said vertical stop.

25. The attachments of claim 24, wherein:
said vertically extending flanges have secondary flanges extending therefrom toward said back portion of said first metal member forming glue pockets adapted to receive glue for gluing said cap to said first metal member.

26. The attachments of claim 24, wherein:
said front portion of said first metal member has a vertical alignment hole extending therein from an upper surface thereof.

27. The attachments of claim 24, wherein said cap has a slot extending horizontally across said front portion thereof at a lower end of said cap.

28. The attachments of claim 24, wherein said back portion of said first metal member comprises horizontally extending tabs defining a means for connecting said first metal member to two tooth engagement members.

29. The attachments of claim 24, wherein first and second metal members of said receptor attachment and said connector attachment are made of stainless steel and are solderable to tooth engagement members.

30. The attachment of claim 24, wherein:
said first metal member of said receptor attachment comprises a front portion defining said connector guide, said connector guide comprising a pair of vertically extending flanges, a vertical stop on said front portion, an intermediate portion extending from said front portion and a back portion extending from said intermediate portion defining a means for connecting to at least one tooth engagement member, said intermediate portion spacing said back portion from said front portion; and
said second metal member of said connector comprises a cap having a front portion defining a means for connecting to an expander assembly, a rear surface engageable with said front portion of said first metal member and channel members on said front portion for engagement with said flanges, said front portion being engageable with said vertical stop.

31. The attachments of claim 30, wherein said vertically extending flanges have secondary flanges extending therefrom toward said back portion of said first metal member forming pockets.

32. The attachment of claim 30, wherein said front portion of said first metal member has a vertical alignment hole extending therein from an upper surface thereof.

33. Attachments for use with an oral osteodistraction device, comprising:
a receptor attachment adapted to be connected with a tooth engagement member of a mandibular distraction device, said receptor attachment comprising a first metal member having a tooth engagement member connection portion and a connector receiving portion; and
a connector attachment adapted to be connected with an expander assembly of a mandibular distraction device, said connector attachment comprising a second metal member comprising an expander assembly connection portion and a receptor connection portion that is engageable with said connector receiving portion of said receptor attachment;
wherein said connector receiving portion of said receptor attachment comprises a connector guide extending thereon;
wherein said receptor connection portion of said connector attachment comprises an engagement surface complementary to said connector guide of said receptor attachment for removable engagement therewith;
wherein said first metal member has a vertical stop thereon that projects outwardly of said connector guide so as to be able to stop said receptor connection portion of said connector attachment; and
wherein said expander assembly connection portion comprises a planar surface facing away from said receptor attachment when said receptor connection portion is engaged with said connector receiving portion.

34. Attachments for use with an oral osteodistraction device, comprising:
a receptor attachment adapted to be connected with an engagement member of an oral osteodistraction device, said receptor attachment comprising a first metal member having an engagement member connection portion and a connector receiving portion; and
a connector attachment adapted to be connected with an expander assembly of the distraction device, said connector attachment comprising a second metal member comprising an expander assembly connection portion and a receptor connection portion that is engageable with said connector receiving portion of said receptor attachment;
wherein said connector receiving portion of said receptor attachment comprises a connector guide extending thereon;
wherein said receptor connection portion of said connector attachment comprises an engagement surface complementary to said connector guide of said receptor attachment for removable engagement therewith; and
wherein said first metal member has a vertical alignment hole extending therein.
